# EUROPEAN PATENT APPLICATION

(11) **EP 0 811 391 A1**
(43) Date of publication of application: **10.12.1997**
(21) Application number: 96109179.0
(22) Date of filing: 07.06.1996
(51) Int. Cl.: A61L 15/46, A61L 15/20

(54) **Absorbent articles having an odour control system comprising a chelating agent**

(71) Applicant: THE PROCTER & GAMBLE COMPANY, Cincinnati, Ohio 45202 (US)
(72) Inventor: Guarracino, Mario, 64028 Silvi Marina (IT); Gagliardini, Alessandro, 60035 Jesi (IT)
(74) Representative: Hirsch, Uwe Thomas

(57) **Abstract**

The present invention relates to an absorbent article comprising an apertured polymeric film topsheet, a backsheet and an absorbent core. The absorbent articles further comprise an odour control system comprising a chelating agent. The combination of the cheating agent and the apertured polymeric film topsheet has been found to provide improved odour control.

## Description

### Field of the Invention

The present invention relates to improved absorbent articles such as diapers, incontinence products, sanitary napkins and panty liners for the absorption of bodily fluids and for the control of the emission of odours from these fluids. The absorbent articles comprise an odour control system comprising a cheating agent in combination with an apertured polymeric film topsheet.

### Background of the Invention

Whilst the primary focus of absorbent articles remains the ability of these articles to absorb and retain fluids, another important area of development in this field is the control of odourous compounds contained within the absorbed fluids or their degradation products. Unpleasant odours which emanate from such articles when in use may cause the wearer to feel self conscious and embarrassed. The detection of such odours from the absorbent article is thus highly undesirable.

As noted above, absorbent articles are well known items of commerce and everyday life. Sanitary napkins and panty liners are typical examples. A sanitary napkin for example normally comprises a topsheet, an absorbent core and a backsheet. The topsheet is that part of the device which in use contacts the body of the user. Fluids discharged from the body normally contact the topsheet and then proceed to pass through it and are retained in the absorbent article by the absorbent core. The topsheet is then normally a porous planar material while the absorbent core is any convenient structure which will absorb fluids. The backsheet prevents the fluids from escaping from the article and soiling the users garments. Absorbent articles comprising an odour controlling agent typically comprise the chemical compound or mixture of compounds having such properties distributed throughout the absorbent core.

There are a wide range of compounds which may be present in an absorbent article during use which result in the formation of malodours. These compounds include fatty acids, ammonia, amines, sulphur containing compounds, ketones and aldehydes. The art is correspondingly replete with descriptions of suitable compounds or agents for use in absorbent articles to address the problem of formation. These compounds or agents can typically be classified according to the type of odour the agent is intended to combat. Odours are typically classified as being acidic, basic or neutral. Acidic odour controlling agents have a pH greater than 7 and typically include inorganic carbonates, bicarbonates, phosphates and sulphates. Basic odour controlling agents have a pH of less than 7 and include compounds such as citric acid, boric acid and maleic acid.

Neutral odour controlling agents have a pH of approximately 7. Examples of these types of compounds include activated carbons, clays, zeolites, silicas and starches and various mixtures thereof as for example described in EPO 348 978, EPO 510 619, WO 91/12029, WO 91/11977 and WO 91/12030. In particular carbon has been noted in the art as being particularly effective over a broad spectrum of odours. However, it is not favoured due to its black appearance, which is considered unacceptable by consumers.

All of the above described types of odour control agents are believed to control odour by mechanisms whereby the malodourous compounds and their precursors are physically absorbed by the agent and thereby hinder the exit of the odour from the absorbent article However, such mechanisms are not completely effective as the formation of the odour itself is not prevented and thus odour detection is not completely avoided.

Hence, there still exists a need to provide an odour controlling agent or system for absorbent articles which provides effective odour control over a wide range of malodourous compounds. In particular the problem of odour control is heightened by the development of thinner and breathable absorbent products.

Surprisingly it has been found that chelating agents in combination with an apertured polymeric topsheet result in a more effective odour controlling absorbent article.

It is believed that the chelating agents link with the metal ions present in the discharge thereby preventing chemical reactions and the formation of odourous degradation products. Furthermore, the chelants also interfere with the microbic metabolism thereby reducing malodour production.

The use of chelants for the reduction of odours has been described in the art, for example US 4 356 190 discloses the use of aminopolycarboxylic compounds and aminophosphonates for inhibiting the production of undesirable products on body surfaces and their use in catamenial products.

EPO 524 581 discloses the removal of odours by the formation of an insoluble complex with the odour causing cations. Suitable compounds include fluorides, phosphates and oxalates.

However, none of the identified prior art recognizes the odour control benefits of the combination of the present invention.

### Summary of the Invention

The present invention relates to an absorbent article comprising an apertured polymeric film topsheet, a backsheet and an absorbent core, the core being intermediate said topsheet and said backsheet. The absorbent article further comprises an odour control system comprising a cheating agent.

### Detailed Description of the Invention

According to the present invention the absorbent articles thus comprise as essential components an apertured polymeric film topsheet and an odour control system comprising a chelating agent and an apertured polymeric film topsheet.

### Odour control system

According to the present invention the odour control system comprises at least one chelating agent as described herein after or mixtures thereof.

### Chelating agents

Suitable chelating agents for use herein can be selected from the group consisting of amino carboxylates, amino phosphonates, polyfunctionally-substituted aromatic chelating agents and mixtures therein, all as hereinafter defined. Without intending to be bound by theory it is believed that the benefit of these materials is in part due to their exceptional ability to remove iron, copper, calcium, magnesium and manganese ions present in the absorbed fluids and their degradation products by the formation of chelates.

Amino carboxylates useful as optional chelating agents include ethylenediamine- tetracetates, N-hydroxyethylethylenediaminetriacetates, nitrilotriacetates, ethylene- diamine tetraproprionates, triethylenetetraaminehexacetates, diethylenetriamine pentaacetates, and ethanoldiglycines, alkali metal, ammonium, and substituted ammonium salts therein and mixtures therein.

Amino phosphonates are also suitable for use as chelating agents in the absorbent articles of the invention and include ethylenediaminetetrakis (methylenephosphonates) as DEQUEST. Preferred, these amino phosphonates do not contain alkyl or alkenyl groups with more than about 6 carbon atoms.

Polyfunctionally-substituted aromatic chelating agents are also useful in the compositions herein. See U.S. Patent 3,812,044, issued May 21, 1974, to Connor et al. Preferred compounds of this type in acid form are dihydroxydisulfobenzenes such as 1,2-dihydroxy-3,5-disulfobenzene.

A preferred biodegradable chelator for use herein is ethylenediamine disuccinate ("EDDS"), especially the [S,S] isomer as described in U.S. Patent 4,704,233, November 3, 1987, to Hartman and Perkins.

Preferably the chelating agent are selected from ethylenediamine-tetracetate, -triacetate, -diacetate, and -monoacetate, N,N, disuccinic acid (sodium salt), ethylenediamine penta (methylene phosponic acid) (sodium salt) ethylenendiamine tetra (methylene phosphonic acid) or mixtures thereof. Most preferably the chelating agent is ethylenediamine tetracetate.

According to the present invention the absorbent articles typically comprise from 5gm⁻² to 300gm⁻², more preferably from 10gm⁻² to 180gm⁻², most preferably from 35m⁻² to 65gm⁻² of said cheating agent.

In addition to the chelating agent the odour controlling system may comprise additional odour control agents known in the art. Suitable agents include zeolites, silica, activated charcoal, antimicrobial agents, absorbent gelling materials or any mixtures thereof. Mixtures of zeolite and silica or zeolite and activated carbon are preferred for use herein.

The chelating agent and any optional odour control agent may be incorporated into the absorbent article by any of the methods disclosed in the art, for example layered on the core of the absorbent article or mixed within the fibres of the absorbent core. The chelating agent is preferably incorporated between two layers of cellulose tissue. Optionally, the system may be bonded between two cellulose tissue layers with, for example, a hot melt adhesive or any suitable bonding system.

More preferably the odour control agent and chelating agent are incorporated in a layered structure in accordance with the disclosure of WO 94/01069 or Italian patent application number TO 93A 001028. TO 93A 001028 describes a layered structure substantially as described in WO 94/01069 with the exception that TO 93A 001028 comprises a much higher quantity of absorbent gelling material (AGM) in the intermediate layer which is between the fibrous layers (120gm⁻²) that would be incorporated as an optional component in the present invention. The intermediate layer comprises in particular a polyethylene powder as thermoplastic material which is mixed with the chelating agent of the present invention and optionally premixed with the optional odour control agent. The mixture is then heated such that the polyethylene melts and glues the laminate layers and components together. The bridges which form the bond points between the fibrous layers involve particles of AGM as well as particles of thermoplastic material. (The absorbent capacity of the AGM is unaffected by bonding.) The adhesive lines are preferably also placed on the edges of the laminate to ensure that the edges of the laminate stick and any loose odour control material or chelating agent does not fall out of the laminate.

The chelating agent and optional odour control agent may be incorporated as a powder or a granulate within the absorbent article. When used in a granulate or particulate form the cheating agent and the odour control agent may be granulated separately and then mixed together or granulated together.

The chelating agent may be distributed homogeneously over the entire absorbent article or in the secondary topsheet or, in at least one layer of the core or any mixture thereof. If additional odour controlling agents are present the chelating agent is positioned such that at least a portion of the fluid discharge comes into contact with said cheating agent before the odour control agent. More preferably the chelating agent is located towards or in the topsheet itself and the odour control material is located further away from the topsheet than the chelating agent, towards the backsheet. In particular, said cheating agent is located in a separate layer from the odour control agent. Most preferably the chelating agent is positioned in at least one of the topsheet layers and the odour control material is positioned in the core.

The chelating agent and optional odor control agent may be distributed homogeneously throughout the absorbent article or any one of the layers thereof, or may be distributed substantially in the centre of the absorbent article or substantially on the edges of the absorbent article.

### Topsheet

According to the present invention the absorbent articles comprise as an essential component an apertured polymeric film topsheet. The term apertured polymeric topsheet as used herein refers to topsheets comprising at least one layer or a multiplicity of layers wherein at least one layer is formed from a continuous or uninterrupted film material wherein apertures are created. It has been surprisingly discovered that apertured polymeric film topsheets yield significantly better odour control than other types of topsheets such as for example thermal bonded nonwoven materials.

In general the apertured polymeric topsheet of the present invention is compliant, soft feeling, and non-irritating to the wearer's skin. Further, the topsheet is fluid pervious permitting fluids (e.g., menses and/or urine) to readily penetrate through its thickness. Suitable apertured polmyeric film topsheets for use herein include polymeric apertured formed films, thermoplastic films, apertured plastic films, and hydroformed thermoplastic films; porous foams; net- like reticulated foams and thermoplastic films; and thermoplastic scrims.

Preferred topsheets for use in the present invention are selected from apertured formed film topsheets. Apertured formed films are especially preferred for the topsheet because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Each of these patents are incorporated herein by reference. Particularly preferred microapetured formed film topsheets are disclosed in U.S. patent 4,609,518 (Curro et al), issue September 2, 1986 and U.S. patent 4,629,643 (Curro et al), issued December 16, 1986, which are incorporated by reference. The preferred topsheet for the present invention is the formed film described in one or more of the above patents and marketed on sanitary napkins by The Procter & Gamble Company of Cincinnati, Ohio as "DRI-WEAVE."

Suitable topsheets in the field of three dimensional formed film are described in EP 0 018 020 and EP 0 059 506. Especially preferred in a three dimensional formed polymeric topsheet having openings in the shape of regular pentagons which are regularly spaced and have an opening of 0.41 square millimeter. The openings are spaced 0.37 square millimeters apart transversely and 0.25 millimeters longitudinally. This topsheet has an initial opening (preforming ) thickness of about 25m a final (post forming) thickness of about 0.53mm and an open area of from 25% to about 40%.

Another formed film topsheet which is especially preferred is one having openings of two shapes; regular pentagons having an area of about 0.21 square millimeters and an irregular hexagon having an area of 1.78 square millimeters. The openings are distributed so that the distance between the sides of the figures is about 0.37 mm to about 0.42mm. The preforming and post forming film thickness are respectively 0.25 and 0.43 mm. This film has an open area of about 33.7%. Both films are made according to the teachings of the above mentioned patents.

A third form suitable topsheet comprises two separate perforated polymeric films superimposed one on the other.

The body surface of the formed film topsheet of the present invention may also be hydrophilic so as to help liquid to transfer through the topsheet faster than if the body surface was not hydrophilic. In this manner the likelihood that menstrual fluid will flow off the topsheet rather than flowing into and being absorbed by the absorbent structure is diminished. In a preferred embodiment, surfactant is incorporated into the polymeric materials of the formed film topsheet such as is described in U.S. Patent Application Serial No. 07/794,745, "Absorbent Article Having A Nonwoven and Apertured Film Coversheet" filed on November 19, 1991 by Aziz, et al., which is incorporated by reference. Alternatively, the body surface of the topsheet can be made hydrophilic by treating it with a surfactant such as is described in the above referenced U.S. 4,950,254, incorporated herein by reference.

### Absorbent core

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent core may have any thickness depending on the end use envisioned.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials in combination with suitable carriers.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### Backsheet

The backsheet primarily prevents the extrudes absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film.

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matt finished to provide a more clothlike appearance. Further, the backsheet can permit vapours to escape from the absorbent structure, i.e. be breathable, while still preventing extrudates from passing through the backsheet. Also breathable backsheets, preferably comprising several layers, e.g. film plus non-woven structures, can be used. Most preferable the backsheet utilises similar or identical material as the topsheet.

According to the present invention the absorbent article may find utility in sanitary napkins, panty liners, adult incontinence products and baby diapers. In particular the present invention finds application in sanitary napkins and panty liners.

### Example:

An Always (Always is a registered Trade Mark) as sold by the Procter & Gamble Company was opened by cutting the wrap around the perforated coverstock at its bottom face approximately along a longitudinal edge of the release paper which covers the external adhesive layer. The side of the absorbent fibrous core is then exposed by slightly shifting the water impermeable plastic bottom layer and subsequently, the fibrous core is split into two halves, each having approximately the same thickness, along a plane which is parallel to the plane of the napkin itself. The cheating agent is homogeneously distributed between these two fibrous layers which are then joined together to reconstitute the absorbent core. The chelating agent used was 0.7-1.0g ethylenediamine tetracetate available from BASF AG.

The water impermeable inner backsheet is then put back into its original position and the wrap around perforated coverstock is sealed along the cut by means of a e.g. a double sided adhesive tape.

## Claims

1. An absorbent article comprising an apertured polymeric film topsheet, a backsheet and an absorbent core, said core being intermediate said topsheet and said backsheet, said article comprising an odour control system comprising a chelating agent.

2. An absorbent article according to claim 1, wherein said chelating agent is selected from aminocaboxylates, aminophosphates, polyfunctionally substituted aromatics or mixtures thereof.

3. An absorbent article according to any one of the preceding claims, wherein said cheating agent is selected from ethylene diamine tetracetate, or N,N, disuccinic acid, or ethylenediamine penta (methylene phosphonic acid), or ethylenediamine tetra (methylene phosphonic acid) or mixtures thereof.

4. An absorbent article according to any one of the preceding claims, wherein said chelating agent is ethylenediamine tetracetate.

5. An absorbent article according to any one of the preceding claims, wherein said absorbent article comprises from 5gm⁻² to 300gm⁻² of said cheating agent.

6. An absorbent article according to any one of the preceding claims, wherein said topsheet is a three dimensional formed polymeric film material.

7. An absorbent article according to any one of the preceding claims, wherein said absorbent article is a sanitary napkin or a panty liner.
